Europäisches Patentamt

European Patent Office

Office européen des brevets

(19)

(11) Publication number: **0 210 924 B1**

# EUROPEAN PATENT SPECIFICATION

(12)

(45) Date of publication of patent specification: 25.09.91    (51) Int. Cl.⁵: **A61B 5/022**, F04B 39/00

(21) Application number: 86401650.6

(22) Date of filing: 23.07.86

(54) Automatic sphygmomanometer.

(30) Priority: 25.07.85 JP 112941/85 U

(43) Date of publication of application:
04.02.87 Bulletin 87/06

(45) Publication of the grant of the patent:
25.09.91 Bulletin 91/39

(84) Designated Contracting States:
BE DE FR GB NL

(56) References cited:
EP-A- 0 014 085
EP-A- 0 106 414
GB-A- 2 060 175

COMPRESSED AIR MAGAZINE, vol. 76, no. 7,
July 1971, pages 7-11, Phillipsburg, US; G.M.
DIEHL: "Think quiet": Part VI- Controlling the
generation of noise"

(73) Proprietor: TERUMO KABUSHIKI KAISHA trad-
ing as TERUMO CORPORATION
44-1, 2-chome, Hatagaya Shibuya-Ku
Tokyo 151(JP)

(72) Inventor: Ide, Tetsuya
361-8, Nakajima-cho
Fujinomiya-shi Shizuoka-ken(JP)
Inventor: Hata, Hideo
Fujimiryo, 2517 Ohmiya
Fujinomiya-shi Shizuoka-ken(JP)

(74) Representative: Descourtieux, Philippe et al
CABINET BEAU de LOMENIE 55 rue
d'Amsterdam
F-75008 Paris(FR)

## Description

This invention relates to an automatic sphygmomanometer having an internal pneumatic pump used in pressurizing a cuff wrapped around an arm of a patient when measuring blood pressure.

In conventional automatic sphygmomanometers having a pneumatic pump, the pump produces noise and vibration when operating. In hospital rooms where silence is required, the use of such a sphygmomanometer poses problems. In particular, it is normally desired that a patient be in a calm mental condition when undergoing a blood pressure measurement. This requires that care be taken to prevent noise and vibration that might otherwise agitate the patient.

To reduce vibration produced by the pneumatic pump during operation, a conventional approach is to support the pump by means such as rubber vibration insulator such as disclosed in GB-A-2 060 175. However, since the rubber vibration insulator is in mechanical contact with the pump and is itself mechanically joined to the case of the sphygmomanometer, pump vibration is diminished in dependence solely upon the damping factor of the rubber. In particular, since a sphygmomanometer has an overall weight of about 1 kg, it is not possible with the conventional arrangement to absorb the vibration energy because of the mass of the sphygmomanometer, and there is the drawback that the entire sphygmomanometer may resonate due to the vibration set up by the pump.

Another disadvantage with the conventional automatic sphygmomanometers is that measures for preventing leakage of noise produced by the pneumatic pump during operation are unsatisfactory. Since noise and vibration can cause anxiety in a patient undergioing blood pressure measurement, the measured values of blood pressure are apt to be abnormal because of these external stimuli. This obviously is an unsatisfactory situation.

The present invention is contrived in the light of the above-mentioned circumstances and an object of the present invention is to provide an automatic sphygmomanometer adapted to prevent vibration and noise produced by a pneumatic pump from being transmitted to the exterior of the sphygmomanometer, thereby minimizing the adverse influence of such stimuli on blood pressure measurement.

According to the present invention, the foregoing object is attained by providing an automatic sphygmomanometer in which pump noise absorbing, vibration absorbing and supporting means comprises an elastomeric body shaped to receive the pump therein, the pump being accommodated within the compartment in a state where the entirety of the pump is snugly enclosed within the elastomeric body; and the pump includes means for generating compressed air for supply to the cuff while the pump is positioned in said state.

Other features and advantages of the present invention will be apparent from the following description taken in conjunction with the accompanying drawings, in which like reference characters designate the same or similar parts throughout the figures thereof.

Fig. 1 is a perspective view schematically illustrating the arrangement of an embodiment of an automatic sphygmomanometer, which is equipped with a pneumatic pump, in accordance with the present invention;

Fig. 2 is a sectional view illustrating the pneumatic pump accommodated in a compartment; and

Fig. 3 is a sectional view schematically illustrating the manner in which the pneumatic pump is connected to tubing.

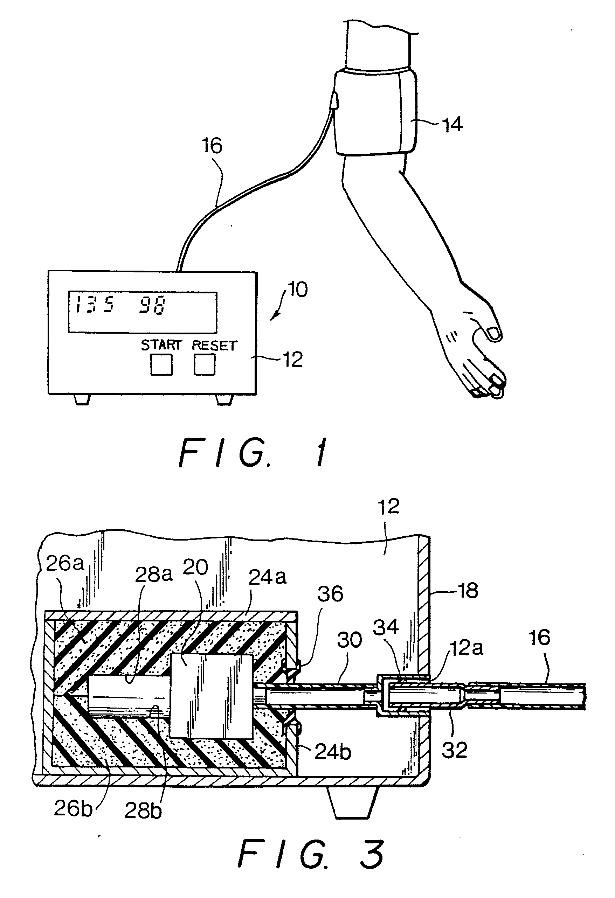

A preferred embodiment of an automatic sphygmomanometer according to the present invention will now be described in detail with reference to Figs. 1 through 3.

With reference to Fig. 1, there is shown an embodiment of an automatic sphygmomanometer 10 comprising a sphygmomanometer instrument package 12, a cuff 14 wrapped around the arm of a patient and serving as a sensor for sensing the patient's blood pressure, and a pipe 16 connecting the instrument package 12 and the cuff 14.

The instrument package 12 is constituted by a detection system for detecting Korotkoff sounds, via the cuff 14, produced by the patient's blood vessels kept under pressure by the cuff, and for determining the patient's blood pressure from the applied pressure and the Korotkoff sounds.

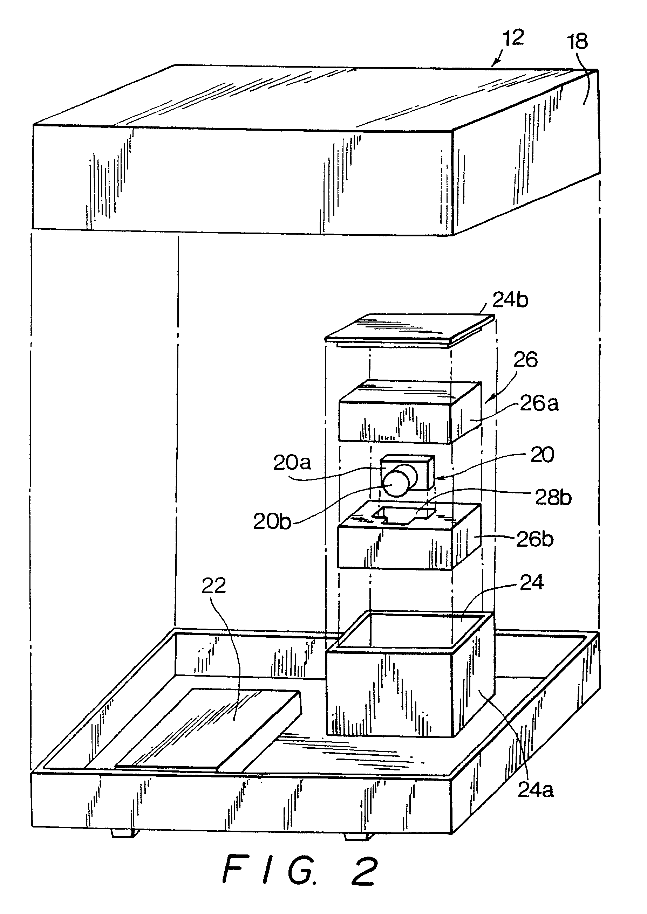

As shown in Fig. 2, the sphygmomanometer instrument package 12 comprises a case 18, a pneumatic pump 20 disposed within the case 18 for feeding compressed air into the cuff 14 via the connecting pipe 16, an arithmetic unit 22 for calculating blood pressure automatically from the Korotkoff sounds sensed by the cuff 14 and the pressure applied to the cuff 14, and a display means for displaying the blood pressure calculated by the arithmetic unit.

More specifically, the case 18 is equipped with a compartment 24 sized to be larger than the pneumatic pump 20 for internally accommodating the same. The pneumatic pump 20 comprises a pump body 20a for emitting compressed air, and a motor 20b for driving the pump body 20a. The motor 20b drives an eccentric shaft, not shown, to move a diaphragm, not shown, up and down, thereby producing compressed air.

The pneumatic pump 20 is accommodated within the compartment 24 in state where the entirety of the pump 20 is covered by a resilient body, such as a rubber foam body 26, serving as resilient means for resiliently supporting the pump 20 within the compartment 24. The rubber foam body 26 has, for example, a density of 15.8 kg/m$^3$. The compartment 24 defines a space delimited by a box 24a open at its top, and an openable lid 24b closing the open top of the box 24a. The rubber foam body 26 comprises first and second halves 26a, 26b capable of being brought together in face-to-face contact. The second half 26b of the rubber foam body 26 is formed to include a recess 28b for receiving approximately the lower half of the pneumatic pump 20. The first half 26a of the rubber body 26 is also formed to include a similar recess 28a (not shown in Fig.2) for receiving approximately the upper half of the pump 20. Thus, when the first and second halves 26a, 26b are brought into face-to-face contact, the pneumatic pump 20 is received snugly in the opposing recesses 28a, 28b, which form a cavity for accommodating the pump. When the rubber foam body 26 is placed in the box 24a, therefore, the entire of the pneumatic pump 20 is accommodated within the compartment 24 in a state where it is completely surrounded by the rubber foam body 26, namely in a state where the pump 20 is not in direct contact with the inner wall of the compartment 24.

As shown in Fig. 3, the compressed air produced by the pneumatic pump 20 is fed into the connecting pipe 16.

Specifically, the case 18 of instrument package 12 is formed to include a recess 12a in its rear side. The recess 12a is connected to the pneumatic pump 20 by a connecting tube 30. Removably inserted in the recess 12a is a connector 32, which is sealed with respect to the peripheral surface of the recess 12a by an O-ring 34. Thus, compressed air is incapable of leaking from between the connector 32 and the case 18. Furthermore, a seal is provided between the connecting tube 30 and the compartment 24 by a thin rubber bushing 36.

Thus, the pneumatic pump 20 is fully enveloped by the rubber foam body 26, in which state the pump is accommodated within the compartment 24. This enables the vibration of the pneumatic pump 26 to be absorbed by the rubber foam body 26 so that the amount of such vibration leaking to the exterior of the instrument package 12 may be suppressed. Also, nearly all the noise from the pneumatic pump 20 is also prevented from leaking to the outside by virtue of the compartment 24, which is sealed by the box 24a and lid 24b.

Thus, in accordance with the embodiment described above, the pneumatic pump 20, which is a source of both noise and vibration, is accommodated within the compartment 24 in a state closely enveloped by the rubber foam body 26, and the compartment 24 is sealed. As a result, external transmission of the noise and vibration ascribable to the pneumatic pump 20 are suppressed.

The invention is not limited to the aforementioned embodiment but can be modified in various ways within the scope of the claims.

For example, though the resilient body is described above as being a rubber foam body, a sponge can also be used.

Since the automatic sphygmomanometer 10 of the invention prevents vibration and noise, the blood pressure detection system is well-suited for application to arrangements that rely upon detection of Korotkoff sounds. However, it goes without saying that the system is not limited to a Korotkoff sound detecting arrangement but can also be applied to a system in which blood pressure is sensed by oscillometry.

According to the present invention as described above, external noise and vibration produced by an automatic sphgymomanometer can be reduced or eliminated through a very simple construction.

## Claims

1. An automatic sphygmomanometer comprising:
   an inflatable cuff (14) to be wrapped around an arm of a patient;
   an instrument body (12) for feeding compressed air into said cuff (14) and for sensing blood pressure by compressing a blood vessel in the arm of the patient via said cuff (14),
   means for connecting said cuff (14) and said instrument body (12) to one another,
   said instrument body (12) including:
   an arithmetic unit (22) for calculating blood pressure;
   a display means for displaying the blood pressure calculated by the arithmetic unit (22),
   a pump compartment (24) having inner surfaces, and a pump (20) therein disposed within said pump compartment; and
   means (26) for absorbing pump vibration and noise, provided between said pump (20) and an inner surface of said compartment (24), and for supporting said pump (20) such that said pump (20) is spaced away from the inner surfaces of said compartment (24);
   characterized in that the pump noise absorbing, vibration absorbing and pump supporting means comprises an elastomeric body (26) shaped to receive said pump (20) therein, said pump (20) being accommodated within said compartment (24) in a state where the entirety of said pump (20) is snugly enclosed within, said elastomeric body (26); and
   said pump (20) includes means for generating compressed air for supply to said cuff (14) while said pump (20) is positioned in said state.

2. An automatic sphygmomanometer according to claim 1, characterized in that said elastomeric body (26) consists of a rubber foam.

3. An automatic sphygmomanometer according to claim 1, characterized in that said elastomeric body (26) consists of sponge.

4. An automatic sphygmomanometer according to any one of claims 1 to 3, characterized in that said compartment (24) is determined by a box (24a) open on one side, and an openable lid (24b) closing the open side of said box (24a).

5. The automatic sphygmomanometer according to claim 4, characterized in that said lid (24b) comprises a discrete member.

6. The automatic sphygmomanometer according to any one of claims 4 and 5, characterized in that said compartment (24) is disposed within a case (18).

**Revendications**

1. Sphygmomanomètre automatique comprenant :

un brassard gonflable (14) destiné à entourer te bras d'un patient ;

un corps d'instrument (12) pour envoyer de l'air comprimé dans ledit brassard (14) et pour capter ta pression artérielle par compression d'un vaisseau sanguin du bras du patient par l'intermédiaire dudit brassard (14) ;

un moyen pour relier l'un à l'autre ledit brassard (14) et ledit corps d'instrument (12) ;

ledit corps d'instrument (12) comprenant :

une unité arithmétique (22) pour calculer la pression artérielle ;

un moyen d'affichage pour afficher la pression artérielle calculée par l'unité arithmétique (22) ;

un compartiment de pompe (24) comportant des surfaces internes, et une pompe (20) disposée à l'intérieur dudit compartiment de pompe ; et

un moyen (26) pour absorber le bruit et les vibrations de ta pompe, disposé entre ladite pompe (20) et une surface interne dudit compartiment (24), et pour soutenir ladite pompe (20) de sorte que ladite pompe (20) est écartée des surfaces internes dudit compartiment (24) ;

caractérisé en ce que le moyen absorbant te bruit de la pompe, les vibrations de la pompe et soutenant ta pompe comprend un corps en élastomère (26) conçu pour recevoir ladite pompe (20), ladite pompe (20) étant logée dans ledit compartiment (24) dans un état où la totalité de ladite pompe (20) est logée à frottement doux dans ledit corps en élastomère (26) ; et

ladite pompe (20) comprend un moyen pour produire de l'air comprimé destiné à alimenter ledit brassard (14) tandis que ladite pompe (20) est placée dans ledit état.

2. Sphygmomanomètre automatique selon ta revendication 1, caractérisé en ce que ledit corps en élastomère (26) consiste en caoutchouc mousse.

3. Sphygmomanomètre automatique selon la revendication 1, caractérisé en ce que ledit corps en élastomère (26) consiste en une matière plastique alvéolaire.

4. Sphygmomanomètre automatique selon l'une quelconque des revendications 1 à 3, caractérisé en ce que ledit compartiment (24) est défini par une boîte (24a) ouverte d'un coté, et par un couvercle amovible (24b) fermant le coté ouvert de ladite boîte (24a).

5. Sphygmomanomètre automatique selon ta revendication 4, caractérisé en ce que ledit couvercle comprend un élément discret.

6. Sphygmomanomètre automatique selon l'une quelconque des revendications 4 et 5, caractérisé en ce que ledit compartiment (24) est disposé à l'intérieur d'un boîtier (18).

**Patentansprüche**

1. Selbsttätiges Sphygmomanometer, umfassend:

eine um einen Arm eines Patienten herumlegbare aufblasbare Manschette (14),

einen Instrumentkörper (12) zum Zuspeisen von Druckluft in die Manschette (14) und zum Messen des Blutdrucks durch Zusammendrükken eines Blutgefäßes im Arm des Patienten über die Manschette (14), (und)

Mittel zum gegenseitigen Verbinden der Manschette (14) und des Instrumentkörpers (12),

wobei der Instrumentkörper (12) aufweist:

eine arithmetische oder Recheneinheit (22) zum Berechnen des Blutdrucks,

eine Anzeigeeinheit zum Anzeigen des durch die Recheneinheit (22) berechneten Blutdrucks,

eine Pumpenkammer (24) mit einer Innenfläche und einer darin innerhalb der Pumpenkammer angeordneten Pumpe (20) sowie

ein zwischen der Pumpe (20) und einer Innenfläche der Kammer (24) angeordnetes Mittel (26) zum Absorbieren von Schwingung und Geräusch der Pumpe (20) und zum Haltern der Pumpe (20) in der Weise, daß die Pumpe (20) auf Abstand von den Innenflächen der Kammer (24) angeordnet ist,

dadurch gekennzeichnet, daß das Mittel zum Absorbieren von Pump(en)geräusch und -schwingung und zum Haltern der Pumpe einen zur Aufnahme der Pumpe (20) (darin) geformten elastomeren Körper (26) umfaßt, wobei die Pumpe (20) in der Kammer (24) in einem Zustand untergebracht ist, in welchem die Gesamtheit der Pumpe (20) passend in den elastomeren Körper (26) eingeschlossen ist, und

die Pumpe (20) eine Einrichtung zum Erzeugen von Druckluft für deren Zuspeisung zur Manschette (14), während die Pumpe (20) in diesem Zustand angeordnet ist, aufweist.

2. Selbsttätiges Sphygmomanometer nach Anspruch 1, dadurch gekennzeichnet, daß der elastomere Körper (26) aus einem Schaumgummi besteht.

3. Selbsttätiges Sphygmomanometer nach Anspruch 1, dadurch gekennzeichnet, daß der elastomere Körper (26) aus Schwammstoff besteht.

4. Selbsttätiges Sphygmomanometer nach einem der Ansprüche 1 bis 3, dadurch gekennzeichnet, daß die Kammer (24) durch einen an der einen Seite offenen Kasten (24a) und einen die offene Seite des Kastens (24) verchließenden, zu öffnenden Deckel (24b) festgelegt ist.

5. Selbsttätiges Sphygmomanometer nach Anspruch 4, dadurch gekennzeichnet, daß der Deckel (24b) aus einem diskreten bzw. getrennten Teil besteht.

6. Selbsttätiges Sphygmomanometer nach einem der Ansprüche 4 und 5, dadurch gekennzeichnet, daß die Kammer (24) innerhalb eines Gehäuses (18) angeordnet ist.

FIG. 1

FIG. 3

F I G. 2